# EUROPEAN PATENT SPECIFICATION

(11) **EP 3 865 137 B1**
(45) Date of publication and mention of the grant of the patent: **25.06.2025**
(21) Application number: 21157648.3
(22) Date of filing: 17.02.2021
(51) Int. Cl.: A61K 36/896, A61K 36/48, A61K 36/87, A61K 31/7048, A61P 9/14, A61K 9/20, A61K 9/48

(54) **COMPOSITIONS AND THEIR USE IN THE TREATEMENT OR PREVENTION OF VENOUS INSUFFICIENCY**
ZUSAMMENSETZUNGEN UND IHRE VERWENDUNG BEI DER BEHANDLUNG ODER VORBEUGUNG DER VENÖSEN INSUFFIZIENZ
COMBINAISONS ET LEUR UTILISATION DANS LE TRAITEMENT OU LA PRÉVENTION DE L'INSUFFISANCE VEINEUSE

(30) Priority: 17.02.2020 IT 202000003122
(43) Date of publication of application: 18.08.2021
(73) Proprietor: ITALFARMACO S.p.A., 20126 Milano (IT)
(72) Inventor: Pizzoni, Angelo, 28041 Arona (NO) (IT); Pizzoni, Paolo, 28041 Arona (NO) (IT)
(74) Representative: Dragotti & Associati S.R.L.

(56) References cited:
- EP-A1- 2 353 596
- IT-A1- MI20 060 802
- IT-A1- RM20 120 591
- CAPASSO F ET AL: "Sistema cardiovascolare", 1 January 2006, FITOTERAPIA: IMPIEGO RAZIONALE DELLE DROGHE VEGETALI, SPRINGER-VERLAG ITALIA, MILANO, PAGE(S) 369 - 424, ISBN: 978-88-470-0505-1, XP008104786
- HOSTETTMANN K ET AL: "ZU INHALTSSTOFFEN UND PHARMAKOLOGIE PFLANZLICHER VENENMITTEL [COMPONENTS AND PHARMACOLOGY OF PHYTOPHARMACEUTIC PREPARATIONS FOR VARICOSE VEINS]", PHLEBOLOGIE, SCHATTAUER GMBH, STUTTGART, DE, vol. 23, no. 3, 1 January 1994 (1994-01-01), pages 71 - 77, XP008104345, ISSN: 0939-978X
- LICHOTA ANNA ET AL: "Therapeutic potential of natural compounds in inflammation and chronic venous insufficiency", EUROPEAN JOURNAL OF MEDICINAL CHEMISTRY, vol. 176, 2019, pages 68 - 91, XP085707318, ISSN: 0223-5234, DOI: 10.1016/J.EJMECH.2019.04.075

## Description

### Summary of the Invention

Subject-matter of the present invention is defined in claims 1-9 and relates to a pharmaceutical/nutraceutical composition and its use in the prevention and/or treatment of venous insufficiency, in particular chronic venous insufficiency, and a process for its preparation.

### Background art

The chronic venous insufficiency (CVI) is shown as stagnant blood in the lower limbs due to the inability of the veins to return it to the heart.

The causes of CVI can be different, for example it can be caused by dilatation of the walls of the veins (varices) or by overwork of the veins, which can result from poor movement or wrong postures. Smoking and excess weight can also lead to the onset of the condition.

The symptoms of CVI comprise the swelling and fatigue of the legs and ankles, the onset of varicose veins, tingling, itching and also pain and cramps that occur mainly during the night. These symptoms tend to occur especially in the summer months and intensify with increased blood stagnation, which causes an increased pressure in the veins, leading in some cases to the rupture of the vessel walls.

There are several compositions containing active ingredients used in the therapy of CVI but they are not always effective.

In the therapy of venous insufficiency and CVI, is becoming increasingly important also the use of products of plant origin that have the ability to modulate the venous flow such as, for example, extracts and essential oils derived from medicinal plants. However, the effectiveness of such natural remedies is often frustrated by the approximation of the dosage, due to the low reliability of the titres of active ingredients in said natural substances, as well as by the fact that the preparation of said natural remedies is generally carried out by the person who takes them, who often does not have the scientific skills necessary to properly manage the treatment.

Therefore, there is a need to provide compositions useful in the treatment and prevention of CVI that overcome the disadvantages of the prior art.

### Objects of the Invention

An object of the present invention is to provide a new combination of active ingredients, having phleboprotective action useful in the treatment and prevention of venous insufficiency and CVI.

Another object of the present invention is to provide a novel composition comprising said combination, particularly suitable for the treatment and/or prevention of CVI.

These and other objects are achieved by the object of the present invention which provides a novel combination of medicinal plants and a composition for the administration thereof.

### Description of the Invention

An subject-matter of the invention, according to one aspect thereof, is a combination consisting of diosmin, Ruscus aculeatus, *Vitis vinifera* and *Melilotus officinalis,* in particular diosmin, extract of Ruscus aculeatus, extract of *Vitis vinifera* and extract of *Melilotus officinalis,* advantageously said extracts being dry extracts.

Diosmin is a molecule belonging to the flavonoid family, whose structural formula is as follows:

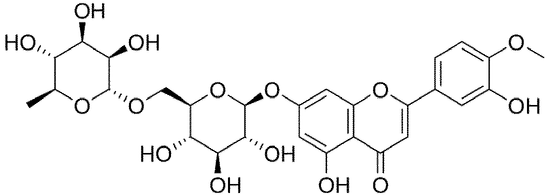

Diosmin is an active ingredient contained in the peel of citrus fruits (genus *Citrus*) from which it can be extracted in combination with hesperidin.

According to a preferred embodiment, in the composition of the invention micronized diosmin, advantageously micronized diosmin with a titre of 90% by weight, is employed. The quantities by weight depicted herein are meant to refer to the weight of diosmin alone. In other words, if 90% by weight micronized diosmin is to be used, when it is depicted that the composition comprises 200 mg diosmin, it is meant that 222.22 mg of 90% by weight micronized diosmin is added to the composition in order to have a diosmin content of 200 mg.

By "ruscus" it is meant herein to denote the dry extract of the roots of *Ruscus aculeatus* (also called butcher's broom), preferably having a ruscogenin content of about 10%.

By *"Vitis vinifera"* it is meant herein a dry extract of the peel of the fruit and seeds (grape pips) of said plant (also known as common vine), preferably having a content of oligomeric proanthocyanidins (OPCs) of more than 80%, preferably around 90-95% and advantageously having a resveratrol content of around 1%.

By "common melilot" it is meant herein to denote a dry extract of *Melilotus officinalis* (also known as yellow sweetclover), preferably having a coumarin content of about 20%.

All active ingredients of the composition of the invention are known and commercially available.

The dried extracts of the medicinal plants of the invention are known in the art and can be prepared with any method useful for the purpose.

Unless otherwise indicated, all percentages in the present description are percentages by weight.

According to the invention, the weight ratios of diosmin/Ruscus *aculeatus*/*Vitis vinifera*/*Melilotus officinalis* are 1/0.2-0.8/0.2-0.6/0.04-0.2, preferably 1/0.3-0.6/0.3-0.5/0.06-0.15, more preferably about 1/0.4/0.4/0.1.

The combination is particularly useful and effective as phleboprotector and to counteract the symptoms and causes of venous insufficiency, in particular CVI. The combination has indeed shown interesting phleboprotective activities thanks to the combined and synergistic action of its components.

The use of the combination as phleboprotector and in the treatment and/or prevention of venous insufficiency, in particular CVI, is a further subject-matter of the invention.

For its use in the treatment and/or prevention of venous insufficiency, in particular CVI, the combination is preferably formulated in compositions suitable for the administration by oral route, with immediate or controlled release, for example in suitable oral dosage forms, such as solid dosage units, such as capsules, tablets, powders, or granules in sachets, or in liquid dosage forms such as mono- or multidose oral solutions or suspensions. Said composition, preferably an oral composition, more preferably an oral solid composition, advantageously with immediate release, comprising the combination of the invention together with pharmaceutically acceptable excipients and carriers, is a further subject-matter of the invention.

In addition to the active ingredients, the compositions of the invention may contain conventionally used excipients, for example diluting agents, adsorbents, lubricants, glidants, sweeteners, colorants, etc., for example selected from glycerol dibehenate, microcrystalline cellulose, magnesium stearate, dicalcium phosphate and silicon dioxide.

With respect to the intake of supplements such as, for example, those in the form of herbal teas, decoctions and similar commercially available natural products, the combination of the invention, in which the active ingredients of plant origin present are preferably titrated as suggested and dosed in pharmaceutical and/or nutraceutical compositions, has the advantage of offering an effective therapy ready for use and carefully calibrated in order to obtain the desired effect.

The combination of the invention may be administered in compositions comprising one or more pharmaceutically acceptable excipients, in daily dosages of:
- from 300 to 1000 mg of diosmin, preferably from 400 to 700, more preferably about 500 mg;
- from 100 to 400 mg of Ruscus aculeatus, preferably from 150 to 300 mg, more preferably about 200 mg;
- from 100 to 300 mg of *Vitis vinifera,* preferably from 150 to 250 mg, more preferably about 200 mg;
- from 20 to 100 mg of *Melilotus officinalis,* preferably from 30 to 70 mg, more preferably about 50 mg;
when said active ingredients are in the form of dry extracts, they preferably have the above titres.

If desired or necessary, e.g., if the doctor deems it useful, such daily dosages may be increased or reduced.

Said dosages can be administered in a single dose or divided in two or more administrations, for example one, two or three daily administrations, to be taken simultaneously or at different times.

According to a preferred embodiment, the combination is contained in oral solid compositions of the capsule, sachet or tablet type.

The compositions of the invention can be defined as pharmaceutical or nutraceutical compositions.

For the preparation of the preferred solid oral composition according to the invention, for example a capsule or a tablet, it is possible to use the methods known in the art. The tablets and capsules may for example be filmed, with cosmetic or functional coatings for example to achieve a gastro-resistant form or a controlled/sustained release.

According to a preferred embodiment, the compositions of the invention are administered orally on an empty stomach and preferably in the morning.

The expression "sustained-release" is intended herein to denote a composition from which the active ingredients are released in a gradual and predetermined way over time.

Such techniques are well known in the field of the pharmaceutical technology.

Some examples of compositions according to the invention are provided in the Experimental Section, by way of illustration.

The use of the composition as described above to treat and/or prevent venous insufficiency, in particular chronic venous insufficiency, is a further subject-matter of the invention.

According to another of its aspects, subject-matter of the invention is a method for obtaining a phleboprotector effect and/or for treating and/or preventing venous diseases such as venous insufficiency, in particular chronic venous insufficiency, which comprises administering an effective dose of the combination or composition of the invention to a subject in the need thereof. By subject is meant herein to denote a mammal, preferably a human.

Although the composition of the invention itself provides the solution to the problem submitted, if desired or needed, other natural extracts and/or other substances however beneficial for the body can be added to the compositions of the present invention, provided that they do not counteract the effects of the combination and composition of the invention.

The invention will now be described in the Experimental Section below for illustrative purpose only and in no way limiting.

### Experimental section

### Example 1

Immediate release, filmed tablet, comprising:
diosmin 500 mg;
Ruscus aculeatus 200 mg;
*Vitis vinifera* 200 mg;
melilot 50 mg;
Ruscus aculeatus, melilot and *Vitis vinifera* being titrated as stated above, together with conventional vehicles and excipients.

### Example 2

Immediate release, filmed tablet, comprising:
diosmin 500 mg;
Ruscus aculeatus 200 mg;
*Vitis vinifera* 200 mg;
melilot 50 mg;
Ruscus aculeatus, melilot and *Vitis vinifera* being titrated as stated above,
together with conventional vehicles and excipients selected from glycerol behenate, microcrystalline cellulose, magnesium stearate, silicon dioxide, dicalcium phosphate.

### Example 3

Hard gelatin capsule comprising:
diosmin 300 mg;
Ruscus aculeatus 150 mg;
*Vitis vinifera* 150 mg;
melilot 30 mg;
Ruscus aculeatus, melilot and *Vitis vinifera* being titrated as stated above, together with conventional vehicles and excipients.

### Example 4

Hard gelatin capsule comprising:
diosmin 300 mg;
Ruscus aculeatus 150 mg;
*Vitis vinifera* 150 mg;
melilot 30 mg;
Ruscus aculeatus, melilot and *Vitis vinifera* being titrated as stated above,
together with conventional vehicles and excipients selected from microcrystalline cellulose and colloidal silica.

### Example 5

### Comparison of the combination of the invention with individual active ingredients

A study was carried out in a preclinical model of venous insufficiency and microcirculation impairment, by comparing the activity of the combination of the invention and the individual components. The analysis was performed on hamsters (Mesocricetus auratus) by using an experimental cheek preparation (De Almeida Cyrino et al., Clin Hemorheol Microcirc. 2018; 68 (4): 371-382). The animals were divided into 6 groups as follows:
1) Vehicle (carboxymethylcellulose 1%)
2) Mixture of diosmin + extract of Melilotus officinalis + extract of Vitis vinifera + extract of Ruscus aculeatus
3) Diosmin
4) Extract of Melilotus officinalis
5) Extract of Vitis vinifera
6) Extract of Ruscus aculeatus

The doses were selected based on the previously published preclinical efficacy, a dose ratio of 1/0.1/0.4/0.4 was maintained in the mixture composition for diosmin, extract of Melilotus officinalis, extract of Vitis vinifera and extract of Ruscus aculeatus, respectively. The administration was performed orally, daily for 14 days. Each group consists of 6 animals.

On day 14, the cheek was everted and blocked with five needles in a circle filled with silicone rubber to provide a flat bottom layer and immersed in a continuous wash with a solution for the evaluation through microscope. The following parameters were evaluated:
- Venotonic effect. Measurement of the arteriole and vein diameters.
- Vascular permeability. Macromolecular permeability measurements after iv. injection of FITC-dextran (molecular weight 150,000 Da). The total cheek area (1 cm²) was observed by measuring the number of leakage sites by manual scanning of the total area at selected time intervals after the topical application of histamine.

Because venous diseases are accompanied by increased infiltration of leukocytes and their adhesion to the capillary venules and large veins (Cheatle et al, Br J Dermatol 1991; 125 (3):.288-90), these features were evaluated after the induction of a local ischemia by a sleeve mounted around the cheek and inflated with air to exert a pressure able to reduce the blood flow. For the leukocyte-endothelium interaction, before the ischemia and 30 minutes after the onset of the reperfusion, the leukocytes were stained with an i.v. infusion of rhodamine G and the fluorescent leukocytes adherent to the endothelium or closer to the venular wall were quantified with a UV light microscope.

### Methods

Male Syrian golden hamsters between 8 and 10 weeks of age were used. A 0.2% isoflurane anesthesia was induced and maintained. Throughout the experiment, the body temperature was maintained at 37.5°C. The cheek was slightly everted and blocked with five needles as mentioned above. In this position, the cheek was immersed in a solution that is continuously washed. An incision of the top layer was produced to swing a triangular flap to one side. The exposed area was sectioned at X10-16 under a stereomicroscope, and the fibrous, almost avascular connective tissue covering the vessels was removed with ophthalmic surgical instruments. The dissected part of the cheek was 125-150 microns thick. The cheeks dissected with petechial formations or those without blood flow in all vessels were discarded. The superfusion solution was HEPES-supported HCO₃-buffered saline (composition in mM: NaCl 110.0, KCl 4.7, CaCl₂ 2.0, MgSO 41.2, NaHCO₃ 18.0, HEPES 15.39, and HEPES Na + salt 14.61); the solution temperature was maintained at 36.5°C, and the superfusion rate ranged from 4 ml/min (arteriolar and venular diameters and leukocyte-endothelium interaction) to 6 ml/min (macromolecular permeability). The pH was set to 7.40 through continuous bubbling of solutions with 5% CO₂ in 95% N₂. For the measurements of arteriolar and venular diameters, the preparations were placed under an intravital microscope in which they were allowed to stand for 30 minutes. The measurements were made via video recordings of each of one to four arterioles and venules selected for each preparation. For the measurement of the macromolecular permeability, 30 min after completion of the preparative procedure, FITC-dextran (molecular weight 150,000 Daltons), with a degree of substitution of FITC molecules 2/1,000 glucose molecules in the polysaccharide chain, was administered at a dose of 25 mg/100 g body weight as an i.v. injection of a 5% solution in 0.9% saline. In the total observed area of the cheek, the number of leakage sites (= leaks) was assessed by manual scanning at selected time intervals, i.e., at 2, 5, 7, 10, 15, 20, and 30 min after the start of each topical application of histamine (5 micro M) USA) that lasted 5 min. The local cheek ischemia was achieved by means of a tubular band placed around the cheek, the simple placement does not interfere with the normal blood flow but the intratubular pressure can be rapidly increased by compressing air into it by using a syringe and then can be rapidly decreased. An intratubular pressure of 200-220 mmHg leads to a complete stop of microvascular blood flow within few seconds. For the leukocyte-endothelium interaction, before the ischemia and 30 minutes after the onset of the reperfusion, the leukocytes were stained with an i.v. infusion of rhodamine G and the fluorescent leukocytes adherent to the endothelium or close to the venular wall were quantified by a UV light microscope (Bouskela et al. Am J Physiol 1992; 262:. H478-85; Svensjo et al., Uppsala J Med Sci. 1978; 83: 71 -9; Persson et al, Int J. MICROCIRC Clin Exp. 1985; 4: 257 -63; Bouskela et al, J. Vasc Res 1999; 36 (suppl 1): 11-14). All results were expressed as mean ± SEM and the analysis of the variance was performed by one- or two-way ANOVA followed by Bonferroni test.

## Claims

1. A combination of active ingredients consisting of diosmin, Ruscus aculeatus, *Vitis vinifera* and *Melilotus officinalis,*
**characterized in that** the weight ratios of diosmin/Ruscus *aculeatus*/*Vitis vinifera*/*Melilotus officinalis* are 1/0.2-0.8/0.2-0.6/0.04-0.2, preferably 1/0.3-0.6/0.3-0.5/0.06-0.15, more preferably about 1/0.4/0.4/0.1.

2. The combination according to claim 1, **characterized in that** it is suitable to be administered in daily dosages of:
- from 300 to 1000 mg of diosmin, preferably from 400 to 700, more preferably about 500 mg;
- from 100 to 400 mg of Ruscus aculeatus, preferably from 150 to 300 mg, more preferably about 200 mg;
- from 100 to 300 mg of Vitis vinifera, preferably from 150 to 250 mg, more preferably about 200 mg;
- from 20 to 100 mg of *Melilotus officinalis,* preferably from 30 to 70 mg, more preferably about 50 mg.

3. The combination according to claim 1 or 2, for its use as phleboprotector and/or in the treatment and/or the prevention of venous insufficiency and chronic venous insufficiency (CVI).

4. A pharmaceutical or nutraceutical composition comprising the combination according to any one of claims 1 to 2 together with one or more pharmaceutically acceptable excipients and carriers.

5. The composition according to claim 4, **characterized in that** it is an oral, preferably solid composition.

6. The composition according to claim 5, **characterized in that** it is a tablet or a film-coated capsule.

7. The composition according to claim 5 or 6, **characterized in that** it is an immediate-release composition.

8. The composition according to claim 5 or 6, **characterized in that** it is a controlled-release composition.

9. The composition according to any one of claims 4 to 8, for its use as phleboprotector and/or in the treatment and/or the prevention of venous insufficiency and chronic venous insufficiency (CVI).

## Patentansprüche

1. Kombination von aktiven Wirkstoffen, bestehend aus Diosmin, Ruscus aculeatus, *Vitis vinifera* und *Melilotus officinalis,*
**dadurch gekennzeichnet, dass** die Gewichtsverhältnisse von Diosmin/Ruscus *aculeatus*/*Vitis vinifera*/*Melilotus officinalis* 1/0,2-0,8/0,2-0,6/0,04-0,2, vorzugsweise 1/0,3-0,6/0,3-0,5/0,06-0,15, besonders bevorzugt etwa 1/0,4/0,4/0,1 betragen.

2. Kombination nach Anspruch 1, **dadurch gekennzeichnet, dass** sie zur Verabreichung in folgenden Tagesdosen geeignet ist:
- 300 bis 1000 mg Diosmin, vorzugsweise 400 bis 700, besonders bevorzugt etwa 500 mg;
- 100 bis 400 mg Ruscus aculeatus, vorzugsweise 150 bis 300, besonders bevorzugt etwa 200 mg;
- 100 bis 300 mg Vitis vinifera, vorzugsweise 150 bis 250 mg, besonders bevorzugt etwa 200 mg;
- 20 bis 100 mg *Melilotus officinalis,* vorzugsweise 30 bis 70 mg, besonders bevorzugt etwa 50 mg.

3. Kombination nach Anspruch 1 oder 2 zur Verwendung als Phleboprotektor und/oder zur Behandlung und/oder Vorbeugung von venöser Insuffizienz und chronischer venöser Insuffizienz (CVI).

4. Pharmazeutische oder nutrazeutische Zusammensetzung, umfassend die Kombination nach einem der Ansprüche 1 bis 2
zusammen mit einem oder mehreren pharmazeutisch verträglichen Exzipienten und Trägern.

5. Zusammensetzung nach Anspruch 4, **dadurch gekennzeichnet, dass** es sich um eine orale, vorzugsweise feste Zusammensetzung handelt.

6. Zusammensetzung nach Anspruch 5, **dadurch gekennzeichnet, dass** sie eine Tablette oder eine Filmkapsel ist.

7. Zusammensetzung nach Anspruch 5 oder 6, **dadurch gekennzeichnet, dass** sie eine Zusammensetzung mit sofortiger Freisetzung ist.

8. Zusammensetzung nach Anspruch 5 oder 6, **dadurch gekennzeichnet, dass** es sich um eine Zusammensetzung mit kontrollierter Freisetzung handelt.

9. Zusammensetzung nach einem der Ansprüche 4 bis 8 zur Verwendung als Phleboprotektor und/oder zur Behandlung und/oder Vorbeugung von venöser Insuffizienz und chronischer venöser Insuffizienz (CVI).

## Revendications

1. Combinaison de principes actifs constituée de diosmine, de Ruscus aculeatus, de *Vitis vinifera* et de *Melilotus officinalis,* **caractérisée en ce que** les rapports pondéraux de diosmine/Ruscus aculeatus/Vitis *vinifera*/*Melilotus officinalis* sont de 1/0,2-0,8/0,2-0,6/0,04-0,2, de préférence de 1/0,3-0,6/0,3-0,5/0,06-0,15, de manière davantage préférée d'environ 1/0,4/0,4/0,1.

2. Combinaison selon la revendication 1, **caractérisée en ce qu'**elle convient pour être administrée à des doses quotidiennes de :
- de 300 à 1000 mg de diosmine, de préférence de 400 à 700, de manière davantage préférée environ 500 mg ;
- de 100 à 400 mg de Ruscus aculeatus, de préférence de 150 à 300 mg, de manière davantage préférée environ 200 mg ;
- de 100 à 300 mg de Vitis vinifera, de préférence de 150 à 250 mg, de manière davantage préférée environ 200 mg ;
- de 20 à 100 mg de *Melilotus officinalis,* de préférence de 30 à 70 mg, de manière davantage préférée environ 50 mg.

3. Combinaison selon la revendication 1 ou 2, pour son utilisation comme phléboprotecteur et/ou dans le traitement et/ou la prévention de l'insuffisance veineuse et de l'insuffisance veineuse chronique (IVC).

4. Composition pharmaceutique ou nutraceutique comprenant la combinaison selon l'une quelconque des revendications 1 à 2 associée à un ou plusieurs excipients et supports pharmaceutiquement acceptables.

5. Composition selon la revendication 4, **caractérisée en ce qu'**il s'agit d'une composition orale, de préférence solide.

6. Composition selon la revendication 5, **caractérisée en ce qu'**il s'agit d'un comprimé ou d'une capsule pelliculée.

7. Composition selon la revendication 5 ou 6, **caractérisée en ce qu'**il s'agit d'une composition à libération immédiate.

8. Composition selon la revendication 5 ou 6, **caractérisée en ce qu'**il s'agit d'une composition à libération contrôlée.

9. Composition selon l'une quelconque des revendications 4 à 8, pour son utilisation comme phléboprotecteur et/ou dans le traitement et/ou la prévention de l'insuffisance veineuse et de l'insuffisance veineuse chronique (IVC).
